# EUROPEAN PATENT APPLICATION

(11) **EP 3 181 059 A1**
(43) Date of publication of application: **21.06.2017**
(21) Application number: 16204214.7
(22) Date of filing: 15.12.2016
(51) Int. Cl.: A61B 17/04, A61B 17/28, A61B 17/30, A61B 17/34

(54) **SURGICAL DEVICE**

(30) Priority: 17.12.2015 HK 15112479
(71) Applicant: AquaMed Limited, Kowloon Ray, Kowloon (HK)
(72) Inventor: CHEN, Yan Fei, Ma On Shan, N.T. Hong Kong (HK)
(74) Representative: 2K Patentanwälte Blasberg Kewitz & Reichel

(57) **Abstract**

The present invention discloses a surgical device including: an operating lever, which includes two pinching arms that integrate to be a solid tube for pinching the object; an arm hold-down tube, which is a round and hollow tube for containing the said two pinching arms, closing the said two pinching arms for pinching the object; and a puncture tube, which is a round and hollow tube for containing the said two pinching arms and the said hold-down tube, the three tubes form the inter-sleeve structure. The device also includes a connection part for connecting the said puncture tube so that the said puncture tube can connect to the tube with larger diameter to form the puncture stabilizing and balancing tube; and a puncture part, which will protrude or retract via the puncture motion mechanism provided at the said puncture tube, so that the object can be pinched with the said operating lever and the said puncture part will protrude for conducting puncture. The device of the present invention can be used for different minimally invasive surgeries, will not bring about any injury and hazard and can be extensively used for surgeries under the endoscope.

## Description

### Technical Field

The present invention relates to the technical field of a kind of medical operation, specifically, relates to a kind of surgical device for minimally invasive surgery.

### Background

For some surgical operations where visual field is poor or the operation space is confined, especially for the operation under endoscope, fairly many surgical apparatuses usually are required to enter the body cavity for pinching and drawing the tissue or suturing, providing assistance for conducting such operations as tissue isolation, transfixion, fixing, cutting and so on. This needs the pinching apparatus that can puncture the body cavity surface and enter the body cavity. Based on the existing technology, although there are apparatuses that can puncture into the body cavity, only the apparatuses for pinching the suture can be used because of the dangerous puncture sharp end operation. There is not apparatus that can both conduct puncture and pinch the tissue and organ.

The greatest difficult problem is the dangerous puncture sharp end operation. As for the path for the surgical apparatus to enter the body cavity, besides that the surgical apparatus enters the body cavity via the sleeve at the abdominal wall incision under endoscope, the only other one path for the surgical apparatus to enter the body cavity is made via puncture. Thus, such surgical apparatus must have sharp front end in order to puncture and pass through the body cavity wall. However, after the relevant surgical apparatus conducts puncture and enters the body cavity for operation, its sharp front end may bring about the danger that the viscera (such as intestinal canal), blood or never is accidentally pierced. Therefore, such surgical apparatuses with sharp front end available in market cannot be used for pinching the tissue and organ, and are limited to carrying/uptaking the suture into the body cavity. In addition, the operation must be conducted with great prudence, and fairly higher requirements are put forward for the experiences and clinical technical capacity of the operator.

Another difficult problem is leaving-out in uptaking. As for the optimal design available in market, the two arms (pinching arms) opening and stretching at both sides of the suture retract into the round tube (arm hold-down tube) with the elastic force of the spring. Namely, the round tube holds down the two arms in passive way, closes the two arms for pinching the suture inside the pinching arms. However, the problem is that: with such design during practical operation, retracting of the pinching arms will cause displacement deviation for uptaking and result in deviating from the uptaking object (for example, the suture). Such inherent design error can only be made up by operator's properly moving the apparatus forward based on the vision and experience, otherwise the uptaking object may deviate from uptaking scope and uptaking fails. For such leaving-out in uptaking, the operator usually needs to accumulate rich experiences for mastering such operation. In fact, even the operator with rich experiences cannot ensure that each uptaking is successful. Much more for the surgical operation conducted with the 2D image of fiber scope, besides that there shall be fine space and posture sense for resisting the hand-eye error, it shall also need to operate the far end of the long moment arm and properly move such far end forward. Thus, whether each operation can be successful or not is a probability event. So, such leaving-out in uptaking extends the operation time, and also increases the operation risk caused by the dangerous and sharp end.

For operation, there is also a great inconvenience (namely the single suture pinching mode). During operation, after uptaking the suture, the operator needs to pinch the suture tightly (pinch the tight suture) in most cases and conduct suture operation while the suture cannot glide. However, in some cases, it needs to pinch the suture loosely, namely the suture will be sleeved in the apparatus, glide inside the apparatus, but does not escape while the apparatus moves (pinching the loose suture) for meeting the operation need. Currently, there is no tool that can both have the tight suture pinching mode and loose suture pinching mode, the operator encounters great inconvenience during operation, and it will take fairly longer time for operation.

In addition, the puncture nose is not sharp enough for the skin. Before puncture, it usually needs to cut a small incision at abdominal wall with the knife. During operation, operations of puncture and pinching are usually conducted alternately. Generally, the operator needs to shift the finger position for realizing operation switching while conducting operation with one hand. However, the current switching design is not convenient. The operator sometimes can complete such switching operation by virtue of assistance from the other hand. Therefore, the operation for the entire apparatus is fairly complex.

For example, in CN102485174A, a kind of vertebral body forming system puncture assembly for treating vertebral body compression fracture with minimal invasion is disclosed. Such assembly includes the puncture needle and the puncture sleeve outside the puncture needle vial muff coupling. The puncture needle tip protrudes out from the puncture sleeve. At the upper end of the puncture needle, there is a handle for the puncture needle. At the upper end of the puncture sleeve, there is a handle for the puncture sleeve. The handle for the puncture needle and the handle for the puncture sleeve can be locked via the locking mechanism. For that invention, the handle for the puncture needle and the handle for the puncture sleeve can be locked via the locking mechanism, this provides convenience for storage and surgical operation; the snapclose and snapclose groove mutually buckle, the limiting ring is used for position limiting and locking purpose, the locking effect is fine; at the limiting ring, there is a rotating and toggling button. Operation is convenient, great practability is provided.

Although such device and method have made certain improvement, the problems above for the existing technology cannot be resolved better yet.

### Summary

The present invention aims at providing a new surgical device for the technical problems above. Such device can be used for different minimally invasive surgeries, can puncture the body cavity with minimal invasion, can be used for pinching the tissue and organ, providing assistance for conducting such operations as tissue and organ isolation, transfixion, cutting and so on, and will not cause the danger that the tissue, organ, blood or nerve is pierced.

For achieving the purposes above, the present invention uses the following technical scheme:
A kind of surgical device, wherein, the device comprises:
   A operating lever comprises:
      Two pinching arms that integrate to be a solid tube for pinching the object;
      An arm hold-down tube, which is a round and hollow tube for containing the two pinching arms, closing the two pinching arms for pinching the object; and
      A puncture tube, which is a round and hollow tube for containing the two pinching arms and the hold-down tube, the three tubes form the inter-sleeve structure;
      A connection part for connecting the puncture tube, so that the puncture tube can connect to the tube with larger diameter to form the puncture stabilizing and balancing tube; and
      A puncture part, which will protrude or retract via the puncture motion mechanism provided at the puncture tube, so that the object can be pinched with the operating lever and the puncture part will protrude for conducting puncture.

For the surgical device of the present invention, there are the upper left hook and lower right hook at each end of the two pinching arms respectively. In addition, there are the semicircular grooves in these two hooks, and the complete round tube passage will form when these two hooks close.

For the surgical device of the present invention, the two pinching arms will form a triangular clearance after closing, and the clearance will be used as the passage for the suture.

For the surgical device of the present invention, the puncture part comprises the front tube of puncture part, middle chamber of the puncture part and the rear tube of the puncture part. Wherein, the middle chamber is square, and the front tube and rear tube are round.

For the surgical device of the present invention, the front tube of puncture part contains the puncture stabilizing and balancing tube, so that the puncture stabilizing and balancing tube can shuttle inside the front tube of puncture part.

For the surgical device of the present invention, there is a puncture motion mechanism at the puncture part. The puncture motion mechanism comprises the device for forward puncture motion, two puncture rotating wheels and the device for backward puncture motion, so that the puncture part can move forward or automatically move backward.

For the surgical device of the present invention, the two puncture rotating wheels are connected via the connecting tube of central axis of the rotating wheels. Wherein, the connecting tube of central axis of the rotating wheels has a four-way structure for stabilizing the motion of the rotating wheels.

For the surgical device of the present invention, part of the arm hold-down tube and pinching arm is wrapped by an external sleeve 20 and an internal sleeve 21 arranged inside the external sleeve 20. Wherein, there is clearance between the external sleeve 20 and the internal sleeve 21, and one end of the external sleeve 20 is connected to the base 25.

For the surgical device of the present invention, the device further comprises a push rod connected to the puncture tube. The push rod comprises the pusher nose having its one end connected to a push rod restricting strip, and another end formed as a semicircular groove.

For the surgical device of the present invention, the device further comprises a mode adjusting part, which is comprised of the mode rotating wheel, mode adjusting and controlling rod and mode adjusting and controlling motion body. The mode adjusting and controlling motion body presents the round and hollow shape, and the push rod and pusher nose are provided inside the adjusting and controlling motion body in order to adjust the surgical device to be under suture tightening mode, or suture loosening mode or tissue mode.

The new type surgical device of the present invention has novel structural design, such device can be used for different minimally invasive surgeries, can puncture the body cavity with minimal invasion and carry the suture into the cavity, can uptake the suture, can be used for pinching the tissue and organ, providing assistance for conducting such operations as tissue and organ isolation, transfixion, cutting and so on. The most important feature is that, the puncture sharp end only protrudes while conducting puncture, retracts into the apparatus under other conditions, will not cause the danger that the tissue, organ, blood or nerve is pierced. Such device is applicable for some open operations that require frequently using the suture or some minimally invasive surgeries where visual field is poor and the operation space is confined, especially for the operation under endoscope.

### Brief description of the drawings

For describing the present invention more clearly, in the following, detailed description of the present invention will be given combining the attached drawings, wherein:
Fig. 1 shows the structure schematic drawing of the surgical device of the present invention;
Fig. 1a shows the structure schematic drawing of the operating lever of the surgical device of the present invention;
Fig. 2 shows the structure schematic drawing of the connection part of the surgical device of the present invention;
Fig. 3 shows the structure schematic drawing of the puncture part of the surgical device of the present invention;
Fig. 3a shows the top view of Fig. 3;
Fig. 3b shows the enlarged drawing of position A of Fig. 3;
Fig. 4 shows the structure schematic drawing of the arm hold-down part of the surgical device of the present invention;
Fig. 4b shows the enlarged drawing of position B of Fig. 4;
Fig. 5 shows the structure schematic drawing of the pinching arm of the surgical device of the present invention;
Fig. 6 shows the left view of the mode adjusting part of the surgical device of the present invention;
Fig. 7 shows the top view of the mode adjusting part of the surgical device of the present invention;
Fig. 7a shows the enlarged drawing of position C of Fig. 7; and
Fig.7b shows the enlarged drawing of position D of Fig. 7.

### Detailed description

In the following, the preferred embodiment of the present invention is given combining the attached drawings in order to provide detailed description of the technical scheme of the present invention.

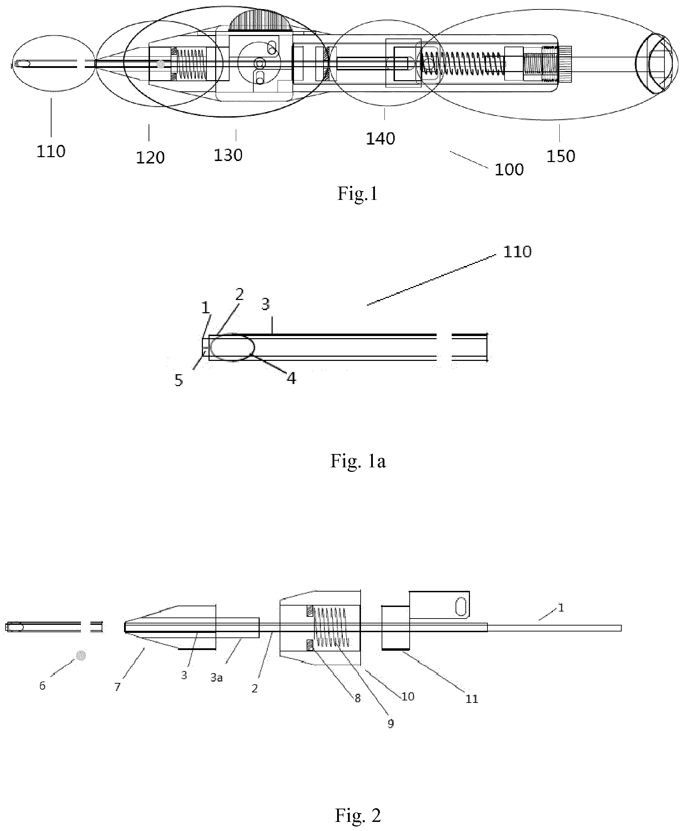

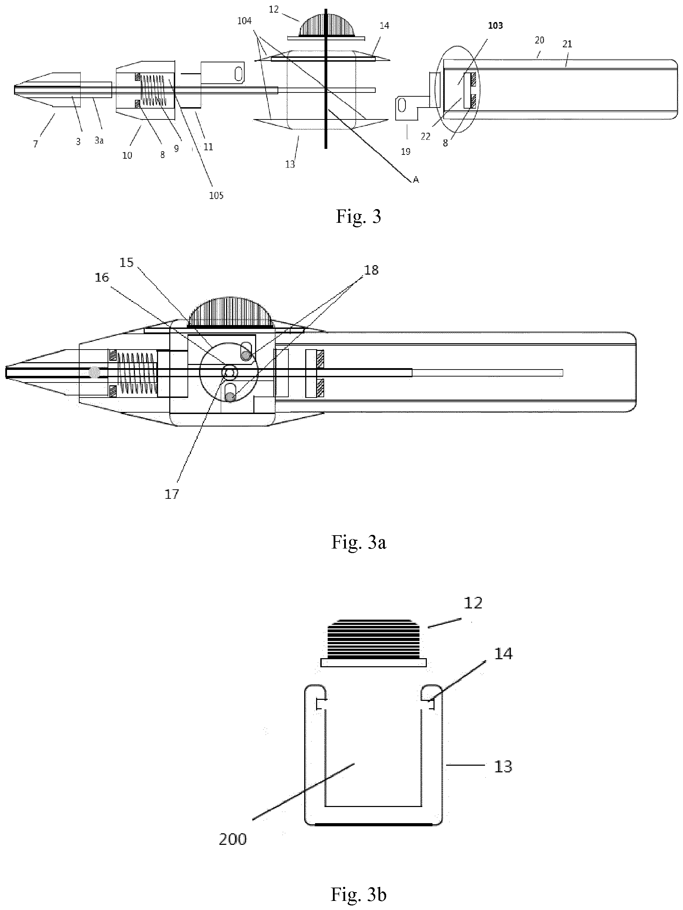

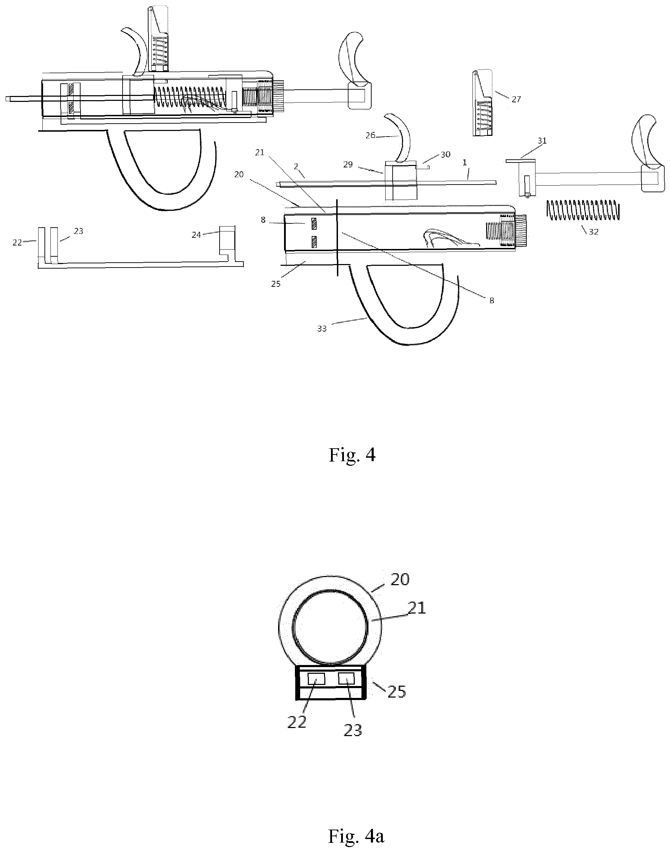

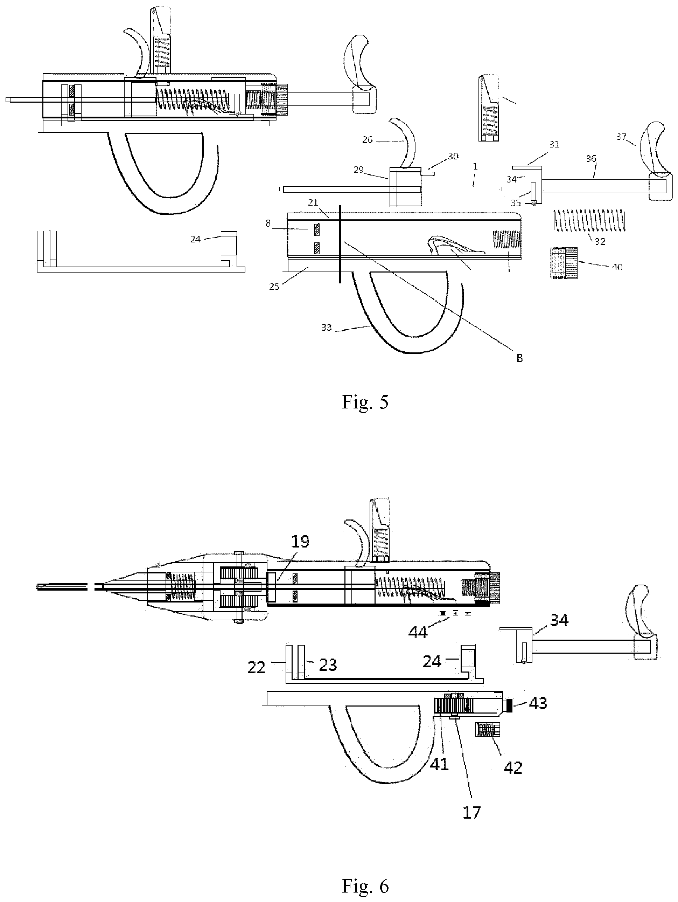

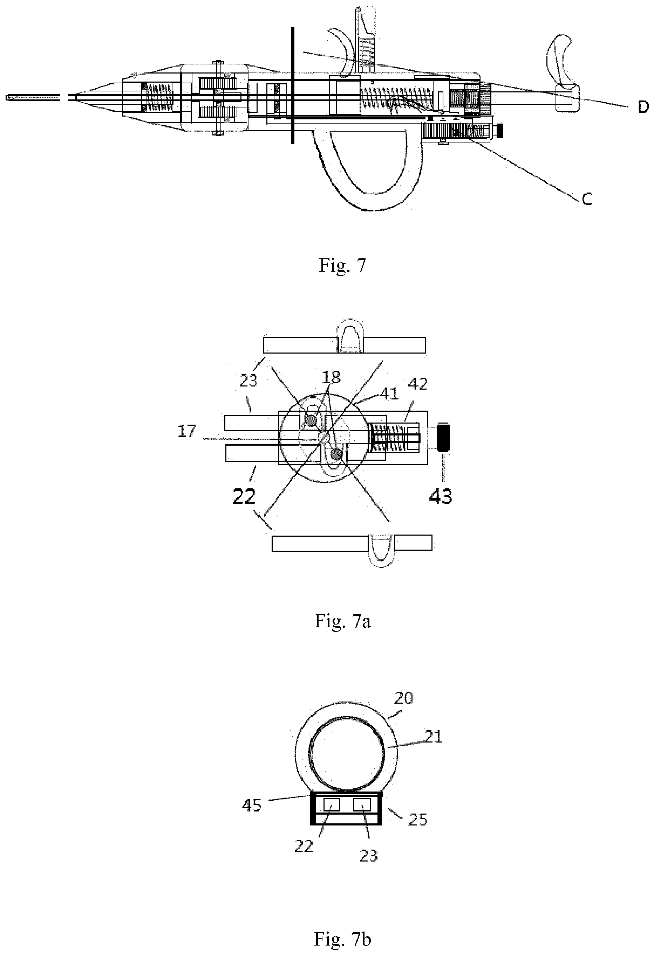

Referring to Figures 1-7b, the surgical device 100 of the present invention comprises: an operating lever 110, which comprises the pinching arm 1 (comprising two pinching arms, the upper pinching arm and the lower pinching arm; these two arms have same length and integrate to be a solid tube for pinching the object involved with the operation); an arm hold-down tube 2, which is a round and hollow tube for containing the pinching arm 1, closing the two pinching arms (upper arm and lower arm) for pinching the object; and a puncture tube 3, which is a round and hollow tube for containing the pinching arm 1 and the hold-down tube 2, the three tubes form the inter-sleeve structure. Namely, the puncture tube 3 contains the arm hold-down tube 2 and pinching arm 1, and the pinching arm 1 is inside the arm hold-down tube 2. The puncture part has a puncture bevel 4.

Further, the two arms have the same length. The upper pinching arm can be made of nickel-titanium memory metal or stainless steel and has plastic elasticity. When the upper pinching arm is pressed and closed by the arm hold-down tube 2 and is then released, the upper pinching arm will restore because of the plastic elasticity and form an angle between the lower pinching arm, the optimal value is about 30°. At rear part, the two arms integrate to be solid round tube, which extends backward and connects to the pusher nose. Thus, when the push rod moves, the pinching arm 1 will move with the push rod.

Preferably, the pinching arm 1 has two small hooks. Within such hooks, there are semicircular grooves, which can form a complete round tube passage when these hooks close (such passage is not shown in Figures). This design can effectively prevent the suture or tissue from leaving out and the suture stays in the suture gliding hole while conducting pinching operation. The suture gliding hole diameter is about 0.4mm. In addition, when two arms close, a triangular hole clearance 5 will form at the sharp end at the upper part of the round tube, such clearance is designed specially as the passage for suture to pass through while conducting puncture and carrying the suture. While carrying the suture, the suture shall wrap and wind up the upper pinching arm, and the longer end of the suture shall be above the upper pinching arm, and the shorter end is below the upper pinching arm. After two arms close and are held down by the arm hold-down tube, the longer end of the suture will be pushed to be inside the triangular hole clearance 5 at above position. Such longer end can be winded tightly at the suture winding nail. In this way, while conducting puncture, the puncture tube sharp bevel will not exceed the above triangular hole clearance, and thus the longer end of the suture will not be cut off, and the shorter end will go out from the suture gliding hole.

The surgical device 100 comprises a connection part 120 for connecting the puncture tube 3, so that the puncture tube 3 can connect to the tube with larger diameter to form the puncture stabilizing and balancing tube 7. Because the puncture tube 3 is very fine and has thin wall, and it is not easy for force exerting, or is easily damaged with force. Thus, puncture tube 7 firstly connects to the extension tube (with larger pipe diameter) of the puncture tube, and the latter solidly connects to the larger round tube to form the puncture stabilizing and balancing tube 7. While conducting puncture, the puncture stabilizing and balancing tube 7 can move within the front tube cavity of the puncture part and have the effects for stabilizing and bearing force in balanced way. The extension tube of the puncture tube leaves from the puncture stabilizing and balancing tube 7, moves backward, enters the front tube cavity of the puncture part and connects to the puncture forward linkage device, the puncture forward motion device connects to the bottom of the push bottom in order to complete the linkage between the puncture tube and the push bottom. The front tube of the puncture part 120/130 comprises several tubes for containing the puncture stabilizing and balancing tube; through welding, the front tube is solidly and structurally connected to the middle chamber of the puncture part.

The surgical device 100 comprises a puncture part 130, which will protrude or retract via the puncture motion mechanism provided at the puncture tube 3, so that the object can be pinched with the operating lever and the puncture part will protrude for conducting puncture. Further, the puncture part comprises the front tube of puncture part, middle chamber of the puncture part and the rear tube of the puncture part. Wherein, the middle chamber is square, and the front tube and rear tube are round. Wherein, the front tube of puncture part contains the puncture stabilizing and balancing tube, so that the puncture stabilizing and balancing tube can shuttle inside the front tube of puncture part.

Preferably, the front tube of puncture part contains the puncture stabilizing and balancing tube 7, there is a spring in front of the puncture forward linkage device. Thus, when force is not exerted via the push button, the puncture tube is compressed inside with elasticity and will not protrude forward, and the safety is improved. The core assembly for mechanical operation of the puncture part 130 comprises the puncture forward linkage device 11, two puncture rotating wheels 15 and the puncture backward linkage device 19. While conducting puncture and exerting force on the push button (the force can only be exerted forward), the linkage device 11 moves forward and makes the puncture tube 3 to protrude forward; the large hole at 11 sheaths the small convex column 18 of the rotating wheel at the external surface, and 15 can also move together, the lower 18 at the internal surface sheaths 19 and makes 19 move backward. At that time, if the mode adjusting and controlling rod 22 inside the rear tube of the puncture part has moved forward, and such rod 22 will be pressed by 19 moving backward to the original position; at the same time, the mode adjusting and controlling rod 22 moves backward with pressure, via the rotating wheels 15, the mode adjusting and controlling motion body 24 will restore to the original setting, the pusher nose moves backward, and thus the pinching arms move backward and restore. The mode adjusting and controlling rod 23 moves together with the mode rotating wheel 41, moves forward and restores to the original place, and the automatic resetting function is completed while conducting puncture.

Further, the connecting tube 16 of central axis of the rotating wheels has a four-way structure, connects to the central axes of the left and right rotating wheels and stabilizes the motion of rotating wheels 15; the arm hold-down tube and the pinching arms pass through the connecting tube 16 of central axis of the rotating wheels and extends backward. The internal sleeve 21 of the rear tube cavity of the puncture part correspondingly has the wall holes for the mode adjusting and controlling rods 22 and 23.

The surgical device 100 comprises the arm hold-down part 140 and the pinching part 150. The main body of the arm hold-down part 140 and the pinching part 150 are jointly wrapped by an external sleeve 20 and an internal sleeve 21 arranged inside the external sleeve 20. There is clearance between the internal and external sleeves 20, 21. The lower part of the external sleeve 20 is cut off at the position even to the lower edge of the internal sleeve 21 and then connects to the base 25; inside the base 25, there is a passage for containing the mode adjusting and controlling rods 22 and 23. Base 25 is connected to the buckle at the low position. End of base 25 connects to the pinching mode part.

The arm hold-down part 140 comprises the pulling hook 26 and gliding ring 29. The pulling hook 26 and gliding ring 29 move together with the arm hold-down tube, and can keep stable. The drive for their linkage mainly comes from two sources: one source is the force when the front spring 32 of the pusher nose is pushed and pressed, and the other source is force when the pulling hook 26 is pulled backward by finger.

At the end of the pulling hook 26, there is an operating button 27 that is used for bearing excessive backward pulling force in case of accident or imprudence.

The linkage system can move only if the operating button 27 is not pressed down, namely, can move only if the arm hold-down tube is operated accurately. At the end of forward motion of the gliding ring 29, there is a mode adjusting and controlling rod 23. If the mode is pinching with loose suture or pinching tissue, the mode adjusting and controlling rod 23 will correspondingly move its position backward, the distance for the gliding ring to move forward will be shortened, namely the length for the arm hold-down tube to protrude forward and close the pinching arms is shortened.

The operating button 27 comprises the casing, button head, button core and spring, and solidly connects to the external sleeve 20. At the position corresponding to the button core, there is a round hole at the internal and external sleeves 20, and such round hole is designed as the passage for the button core to move downward. The casing has fairly thicker front wall, the upper and inside surface is engraved with a tortoise-shape groove, and there is also a transverse groove at front wall of the button head. The groove is the track for a small steel ball, and is sued for controlling the motion of the operating button.

The pinching part 150 and the arm hold-down tube part 140 have overlapped components. The pinching part accounts for the major part of the external sleeve 20 and internal sleeve 21. The core of the pinching part 150 is the round pusher nose 34 in the internal sleeve. The upper part of the pusher nose connects to the limiting strip 31, and there is a groove at the semi-circle of its lower part, there is a steel ball in the groove; in the front, the pusher nose 34 connects to the pinching arm and front spring 32 of the pusher nose. Behind it, the pusher nose connects to the push rod 36 and the rear spring 39 of the pusher nose, and the pusher nose is adjacent to the mode adjusting and controlling motion body 24, and thus the adjusting and controlling motion body 24 can push the pusher nose 34 forward, and drive the pinching arm 1 to move forward.

The push rod limiting strip 31 connects to the pusher nose 34, and can move inside the clearance between the internal and external sleeves. The mode adjusting and controlling motion body 24 is inside the internal sleeve 21, presents the round and hollow structure. At the inside surface of the lower semicircle of the internal sleeve corresponding to the lower semicircle of the pusher nose 34, there is a fan-shape groove 38, the groove 38 is like the fan handle and is located at the center just below the curved surface of the lower semicircle of the internal sleeve. Half of the steel ball is at the pusher nose groove 35, and another half is at such fan handle.

The device further comprises the pinching mode part, which comprises the mode rotating wheel 41, mode adjusting and controlling rods 22 and 23 and mode adjusting and controlling motion body 24. The motion body is a round and hollow structure, where the push rod 36 and the rear spring 39 of the pusher nose can pass. The lower part of the motion body connects to the mode adjusting and controlling rod 22. The adjustable modes of the pinching mode part include three modes, namely the pinching mode with tight suture, pinching mode with loose suture and the tissue pinching mode.

Specifically, shown as the figures, the adjusting and controlling rods 22 and 23 inside the base passage sheath with the small convex column 18 of the rotating wheel. The end of the mode adjusting and controlling rod 22 upward passes through the internal sleeve 21 and connects to the mode adjusting and controlling motion body 24, and there is a hole clearance in front of the mode adjusting and controlling rod 22 corresponding to the internal sleeve 21 and upper plate of the base, so that the mode adjusting and controlling motion body 24 can move forward. When the mode rotating wheel 41 moves forward, the mode adjusting and controlling rod 22 and the mode adjusting and controlling motion body 24 will be driven to move forward , and the mode adjusting and controlling rod 23 will move backward.

While conducting puncture, the device has the automatic resetting function. Thus, the mode adjusting and controlling rod 22 can move backward and restore, the mode adjusting and controlling motion body 24 also moves backward, the mode rotating wheel 41 will be driven to make the adjusting and controlling rod 23 to move forward for completing the restoration action.

The mode rotating wheel 41 connects to the adjusting tube 42 and adjusting button 43 of the mode rotating wheel; the adjusting tube 42 and adjusting button 43 are used for stabilizing the mode rotating wheel 41. The force is screwed and pressed to the mode rotating wheel 41 via the spring of the adjusting button 43, so that the rotating wheel 41 is relatively fixed and is not easy to displace.

Based on the technology above, the new type surgical device of the present invention can be used for different minimally invasive surgeries, can puncture the body cavity with minimal invasion and carry the suture into the cavity, can uptake the suture, can be used for pinching the tissue and organ, providing assistance for conducting such operations as tissue and organ isolation, transfixion, cutting and so on. For the design, the puncture sharp end only protrudes while conducting puncture, retracts into the apparatus under other conditions, will not cause any injury and danger. Such device can be extensively utilized for some open operations that require frequently using the suture or some minimally invasive surgeries where visual field is poor and the operation space is confined, especially for the operation under endoscope.

With the specific embodiment above, the solved technical problem, technical solution and beneficial effects of the present invention are described in further and detailed way. It is understood that, the description above is only a specific embodiment of this present invention, does not intend to limit this present invention. Any modification, equivalent replacement, improvement and so on within the essence and principle of this present invention shall be within the protection scope of this present invention.

## Claims

1. A surgical device (100), comprising:
an operating lever (110) comprising:
two pinching arms (1) formed as a solid tube for pinching an object;
an arm hold-down tube (2), which is a round and hollow tube for containing the two pinching arms, closing the two pinching arms for pinching the object; and
a puncture tube (3), which is a round and hollow tube for containing the two pinching arms and the hold-down tube, forming an inter-sleeve structure of three tubes;
a connection part (120) for connection of the puncture tube, so that the puncture tube could connect to a tube with a larger diameter to form a puncture stabilizing and balancing tube; and
a puncture part (130), which could protrude or retract via a puncture motion mechanism provided at the puncture tube, so that the object can be pinched with the operating lever and the puncture part could protrude for performing puncture operations.

2. The surgical device of the claim 1, wherein, there are an upper left hook and a lower right hook at each end of the two pinching arms respectively; and there are semicircular grooves in these two hooks, and a complete round tube passage will form when these two hooks close.

3. The surgical device of the claim 1 or 2, wherein, the two pinching arms will form a triangular clearance after closing, and the clearance will be used as the passage for the suture.

4. The surgical device of any of the claims 1 to 3, wherein, the puncture part comprises a front tube of puncture part, a middle chamber of the puncture part and a rear tube of the puncture part; wherein, the middle chamber is square, and the front tube and rear tube are round.

5. The surgical device of the claim 4, wherein, the front tube of puncture part contains the puncture stabilizing and balancing tube, so that the puncture stabilizing and balancing tube can shuttle inside the front tube of puncture part.

6. The surgical device of the claim 4 or 5, wherein, there is a puncture motion mechanism at the puncture part; the puncture motion mechanism comprises the device for forward puncture motion, two puncture rotating wheels and the device for backward puncture motion, so that the puncture part can move forward for conducting puncture, or can automatically move backward for retracting the puncture tube.

7. The surgical device of the claim 6, wherein, the two puncture rotating wheels are connected via the connecting tube of central axis of the rotating wheels; wherein, the connecting tube of central axis of the rotating wheels has a four-way structure for stabilizing the motion of the rotating wheels.

8. The surgical device of any of the claims 1 to 7, wherein, part of the arm hold-down tube and pinching arm is wrapped by an external sleeve 20 and an internal sleeve 21 arranged inside the external sleeve 20; wherein, there is a clearance between the external sleeve 20 and the internal sleeve 21, and one end of the external sleeve 20 is connected to the base 25.

9. The surgical device of any of the claims 1 to 8, wherein, the device further comprises a push rod connected to the puncture tube; the push rod comprises a pusher nose having its one end connected to a push rod restricting strip, and another end formed as a semicircular groove.

10. The surgical device of the claim 9, wherein, the device further comprises a mode adjusting part, which comprises a mode rotating wheel, a mode adjusting and controlling rod, and a mode adjusting and controlling motion body; the mode adjusting and controlling motion body has a round and hollow shape, and the push rod and pusher nose are provided inside the adjusting and controlling motion body in order to adjust the surgical device to be under suture tightening mode, or suture loosening mode or tissue mode.
